(19) Europäisches Patentamt
European Patent Office
Office européen des brevets

(11) Veröffentlichungsnummer: **0 392 240 B1**

# (12) EUROPÄISCHE PATENTSCHRIFT

(45) Veröffentlichungstag der Patentschrift: **11.05.94**

(51) Int. Cl.⁵: **C07D 239/28**, C07D 239/34, C07D 405/12, A01N 47/18

(21) Anmeldenummer: **90105655.6**

(22) Anmeldetag: **24.03.90**

(54) **Pyrimidin-4-yl-carbamidsäureester.**

(30) Priorität: **08.04.89 DE 3911488**

(43) Veröffentlichungstag der Anmeldung:
**17.10.90 Patentblatt 90/42**

(45) Bekanntmachung des Hinweises auf die Patenterteilung:
**11.05.94 Patentblatt 94/19**

(84) Benannte Vertragsstaaten:
**BE CH DE DK ES FR GB IT LI NL**

(56) Entgegenhaltungen:
**EP-A- 0 022 511**
**DE-A- 2 752 613**
**DE-A- 2 838 359**
**GB-A- 1 181 657**

(73) Patentinhaber: **BAYER AG**

**D-51368 Leverkusen(DE)**

(72) Erfinder: **Maurer, Fritz, Dr.**
**Roeberstrasse 8**
**D-5600 Wuppertal 1(DE)**
Erfinder: **Findeisen, Kurt, Dr.**
**Dünfelder Strasse 28**
**D-5090 Leverkusen 1(DE)**
Erfinder: **Hartwig, Jürgen, Dr.**
**Franz-Esser-Strasse 44**
**D-5090 Leverkusen 3(DE)**
Erfinder: **Becker, Benedikt, Dr.**
**Metzkausener Strasse 14**
**D-4020 Mettmann(DE)**

**EP 0 392 240 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Pyrimidin-4-yl-carbamidsäureester, mehrere Verfahren zu ihrer Herstellung und ihre Verwendung als Schädlingsbekämpfungsmittel, insbesondere als Insektizide.

Es ist bekannt, daß bestimmte Pyrimidin-4-yl-carbamidsäureester, wie z.B. 2-Dimethylamino-5,6-diethylpyrimidin-4-yl-dimethylcarbamat, insektizid wirksam sind (vgl. GB-A 1181 657). Die Wirkung und Wirkungsdauer dieser Verbindungen sind jedoch, insbesondere bei niedrigen Aufwandmengen bzw. Wirkstoffkonzentrationen, nicht immer voll zufriedenstellend.

Es wurden nun neue Pyrimidin-4-yl-carbamidsäureester der Formel (I) gefunden,

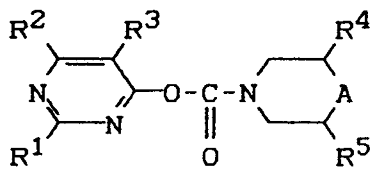

(I)

in welcher

| | |
|---|---|
| $R^1$ | für Alkoxy mit 1 bis 4 Kohlenstoffatomen, Alkylthio mit 1 bis 4 Kohlenstoffatomen, Alkylthioalkyl mit 1 bis 4 Kohlenstoffatomen in den einzelnen Alkylteilen, Alkylsulfinylalkyl mit 1 bis 4 Kohlenstoffatomen in den einzelnen Alkylteilen, Alkylsulfonylalkyl mit 1 bis 4 Kohlenstoffatomen in den einzelnen Alkylteilen und Dialkylamino mit 1 bis 3 Kohlenstoffatomen in den einzelnen Alkylteilen steht, |
| $R^2$ | für Wasserstoff oder Alkyl mit 1 bis 4 Kohlenstoffatomen steht, |
| $R^3$ | für Wasserstoff, Alkyl mit 1 bis 4 Kohlenstoffatomen oder Alkoxy mit 1 bis 4 Kohlenstoffatomen steht oder |
| $R^2$ und $R^3$ | gemeinsam für einen zweifach verknüpften, geradkettigen oder verzweigten Alkandiylrest mit 2 bis 5 Kohlenstoffatomen stehen, |
| $R^4$ | für Wasserstoff oder Alkyl mit 1 bis 3 Kohlenstoffatomen steht, |
| $R^5$ | für Wasserstoff oder Alkyl mit 1 bis 3 Kohlenstoffatomen steht und |
| A | für Sauerstoff, Schwefel oder für einen Rest |

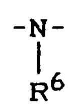

steht, wobei

$R^6$ für Alkyl mit 1 bis 3 Kohlenstoffatomen steht.

Weiterhin wurde gefunden, daß man die neuen Pyrimidin-4-yl-carbamidsäureesterder Formel (I) erhält, wenn man

a) 4-Hydroxy-pyrimidin-Derivate der Formel (II)

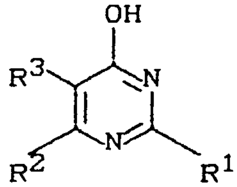

(II)

in welcher

$R^1$, $R^2$ und $R^3$ die oben angegebene Bedeutung haben,

oder das entsprechende Alkalimetall-, Erdalkalimetall- oder Ammoniumsalz

(α) mit Carbamidsäurechloriden der Formel (III)

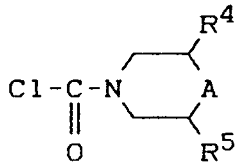

(III)

in welcher

$R^4$, $R^5$ und A die oben angegebene Bedeutung haben,

gegebenenfalls in Gegenwart eines Säureakzeptors und gegebenenfalls in Gegenwart eines Verdünnungsmittels umsetzt oder

(β) mit Phosgen der Formel (IV)

$COCl_2$ (IV)

gegebenenfalls in Gegenwart eines Säureakzeptors und gegebenenfalls in Gegenwart eines Verdünnungsmittels umsetzt

und die sich bildenden Chlorameisensäure-pyrimidylester der Formel (V)

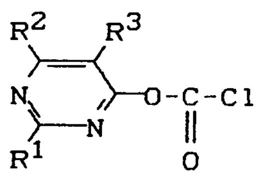

(V)

in welcher

$R^1$, $R^2$ und $R^3$ die oben angegebene Bedeutung haben,

gegebenenfalls nach der Isolierung mit Aminen der allgemeinen Formel (VI)

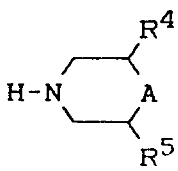

(VI)

in welcher

$R^4$, $R^5$ und A die oben angegebene Bedeutung haben,

gegebenenfalls in Gegenwart eines Säureakzeptors und gegebenenfalls in Gegenwart eines Verdünnungsmittels umsetzt

oder

b) die nach Verfahren (a-α) und (a-β) erhältlichen Pyrimidin-4-yl-carbamidsäureester der Formel (Ia)

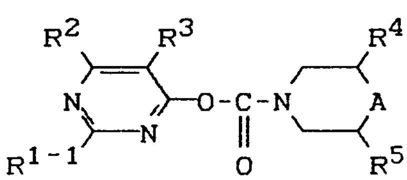

(Ia)

in welcher

$R^2$, $R^3$, $R^4$, $R^5$ und A — die oben angegebene Bedeutung haben und
$R^{1-1}$ — für Alkylthioalkyl mit 1 bis 4 Kohlenstoffatomen in den einzelnen Alkylteilen steht,

mit 1 bis 3,5 Mol eines Oxidationsmittels pro Mol Carbamidsäureester (Ia), gegebenenfalls in Gegenwart von Verdünnungsmitteln und gegebenenfalls in Gegenwart eines Katalysators, umsetzt und die Verbindungen der allgemeinen Formel (I) isoliert.

Die neuen Pyrimidin-4-yl-carbamidsäureester der Formel (I) zeichnen sich in überragender Weise durch eine besonders hohe Wirksamkeit als Schädlingsbekämpfungsmittel, insbesondere als Insektizide, aus.

Die Verbindungen der Formel (I) zeichnen sich außerdem durch eine besonders gute systemische Wirksamkeit aus.

Bevorzugte Substituenten bzw. Bereiche der in den oben und nachstehend erwähnten Formeln aufgeführten Reste werden im folgenden erläutert:
In den allgemeinen Formeln bedeutet Alkyl geradkettiges oder verzweigtes Alkyl mit 1 bis 4 Kohlenstoffatomen; beispielhaft seien Methyl, Ethyl, n- und i-Propyl, n-, i-, s- und t-Butyl genannt.

Alkyl steht weiterhin auch in alkylhaltigen Resten wie Alkylsulfinylalkyl, Dialkylamino, Alkylsulfonylalkyl, Alkoxy, Alkylthio und Alkylthioalkyl, falls nicht anders definiert, für geradkettiges oder verzweigtes Alkyl mit 1 bis 4 Kohlenstoffatomen. Beispielhaft seien genannt: Methoxy, Ethoxy, n- und i-Propyloxy, Methylthio, Ethylthio, n-und i-Propylthio, Methylthiomethyl, Ethylthiomethyl, 2-Methylthioethyl, Methylsulfinylmethyl, Ethylsulfinylmethyl, 2-Methylsulfinylethyl, Methylsulfonylmethyl, Ethylsulfonylmethyl, 2-Methylsulfonylethyl, Dimethylamino oder Diethylamino.

Von den erfindungsgemäßen Pyrimidin-4-yl-carbamidsäureestern der Formel (I) sind diejenigen bevorzugt,
in welchen

$R^1$ — für Methoxy, Ethoxy, Methylthio, Ethylthio, Methylthiomethyl, Ethylthiomethyl, 2-Methylthioethyl, Methylsulfinylmethyl, Ethylsulfinylmethyl, 2-Methylsulfinylethyl, Methylsulfonylmethyl, Ethylsulfonylmethyl, 2-Methylsulfonylethyl, Dimethylamino oder Diethylamino steht,
$R^2$ — für Wasserstoff, Methyl oder Ethyl steht,
$R^3$ — für Wasserstoff, Methyl, Ethyl, Isopropyl, Methoxy oder Ethoxy steht oder
$R^2$ und $R^3$ — gemeinsam für einen zweifach verknüpften Alkandiylrest mit 3 oder 4 Kohlenstoffatomen stehen,
$R^4$ — für Wasserstoff oder Methyl steht,
$R^5$ — für Wasserstoff oder Methyl steht und
A — für Sauerstoff, Schwefel oder für den Rest

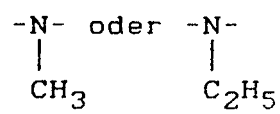

steht.

Im einzelnen seien außer den bei den Herstellungsbeispielen genannten Verbindungen die in der nachfolgenden Tabelle 1 aufgeführten Pyrimidin-4-yl-carbamidsäureester der allgemeinen Formel (I) genannt:

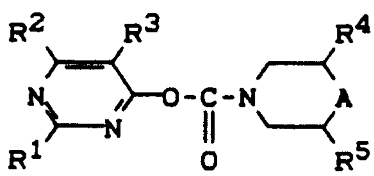

(I)

Tabelle 1

| $R^1$ | $R^2$ | $R^3$ | $R^4$ | $R^5$ | A |
|---|---|---|---|---|---|
| $OCH_3$ | $CH_3$ | $CH_3$ | H | H | O |
| $SCH_3$ | $CH_3$ | $CH_3$ | H | H | O |
| $CH_2CH_2$-$SCH_3$ | H | $OCH_3$ | H | H | O |
| $CH_2CH_2$-$SO_2CH_3$ | H | $OCH_3$ | H | H | O |
| $CH_2CH_2$-$SCH_3$ | $CH_3$ | $CH_3$ | H | H | O |
| $CH_2CH_2$-$SO_2CH_3$ | $CH_3$ | $CH_3$ | H | H | O |
| $CH_2$-$SO_2$-$C_2H_5$ | $CH_3$ | $CH_3$ | H | H | O |
| $CH_2$-$SO_2$-$CH_3$ | $CH_3$ | $C_2H_5$ | H | H | O |
| $CH_2$-$SO_2$-$CH_3$ | $CH_3$ | $C_3H_7$-i | H | H | O |
| $CH_2$-$SO_2$-$CH_3$ | H | $C_3H_7$-i | H | H | O |
| $CH_2$-$SO_2$-$CH_3$ | H | $OC_2H_5$ | H | H | O |
| $CH_2$-$SO_2$-$CH_3$ | -$CH_2CH_2CH_2CH_2$- | | H | H | O |
| $CH_2$-$SO_2$-$CH_3$ | $CH_3$ | $CH_3$ | H | H | O |
| $CH_2$-SO-$CH_3$ | -$CH_2$-$CH_2$-$CH_2$- | | H | H | O |
| $N(CH_3)_2$ | $CH_3$ | $CH_3$ | $CH_3$ | $CH_3$ | O |
| $CH_2$-$SO_2$-$CH_3$ | -$CH_2$-$CH_2$-$CH_2$- | | $CH_3$ | $CH_3$ | O |
| $CH_2CH_2SO_2CH_3$ | H | $C_3H_7$-n | H | H | O |

Verwendet man beispielsweise 2-Methylsulfonylmethyl-4-hydroxy-5-methoxypyrimidin und Morpholinocarbamidsäurechlorid als Ausgangsstoffe, so kann das erfindungsgemäße Verfahren (a-$\alpha$) durch das folgende Formelschema skizziert werden:

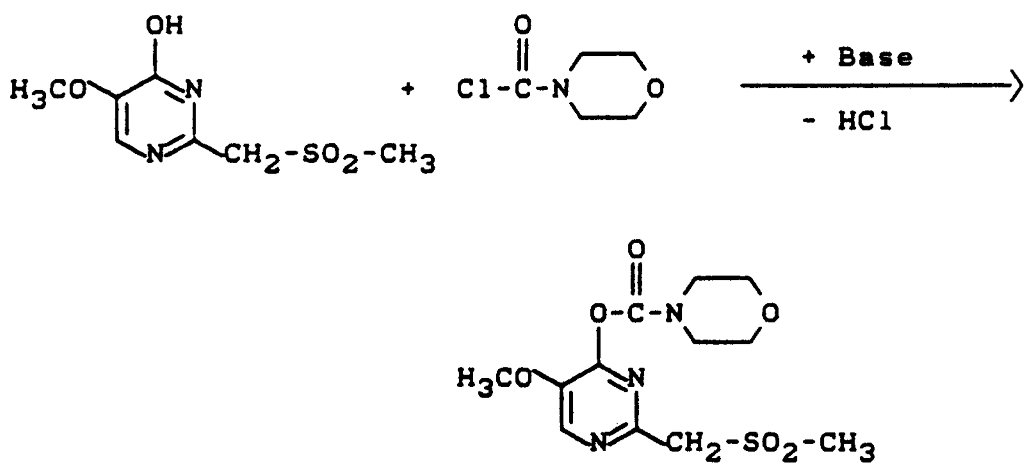


Verwendet man beispielsweise 2-Methylsulfonylmethyl-4-hydroxy-5,6-dimethylpyrimidin, Phosgen und Dimethylmorpholin als Ausgangsstoffe, so kann das erfindungsgemäße Verfahren (a-$\beta$) durch das folgende Formelschema skizziert werden:

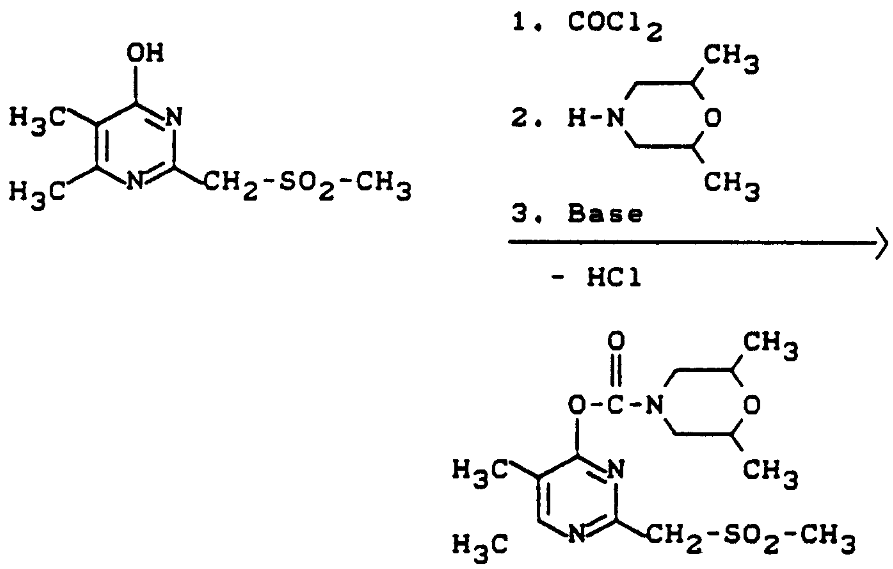


Verwendent man beispielsweise O-(2-Methylthiomethyl-5,6-dimethyl-pyrimidin-4-yl)-morpholinocarbamidsäureester als Ausgangsstoffe und Wasserstoffperoxid in Gegenwart von katalytischen Mengen Ammoniummolybdat als Oxidationsmittel, so kann das erfindungsgemäße Verfahren (b) durch das folgende Formelschema skizziert werden:

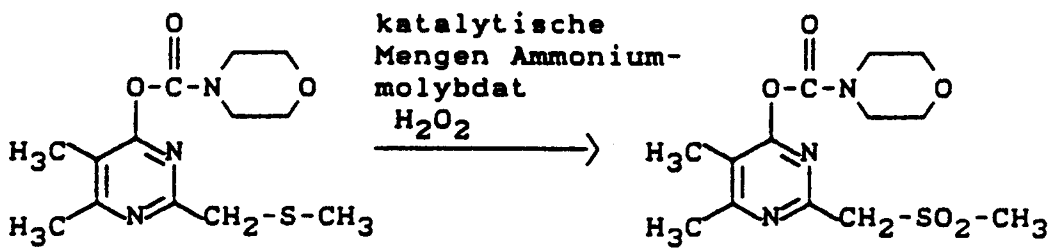


Die bei den erfindungsgemäßen Verfahren (a-α) und (a-β) als Ausgangsstoffe für die Herstellung der neuen Pyrimidin-4-yl-carbamidsäureester der Formel (I) einzusetzenden 4-Hydroxy-pyrimidin-Derivate bzw. die entsprechenden Alkalimetall-, Erdalkalimetall- oder Ammoniumsalze sind durch die Formel (II) definierte. In dieser Formel stehen $R^1$, $R^2$ und $R^3$ für diejenigen Reste, die oben bei der Definition in Formel (I) angegeben sind. Als Alkali- oder Erdalkalimetallsalze werden vorzugsweise die Natrium-, Kalium- oder Calciumsalze eingesetzt.

Die Verbindungen der Formel (II) sind bekannt und/oder können nach allgemein bekannten Verfahren und Methoden hergestellt werden (vgl. z.B. DE-A 34 45 465 und DE-A 33 24 399).

Die bei dem erfindungsgemäßen Verfahren (a-α) außerdem als Ausgangsstoffe einzusetzenden Carbamidsäurechloride sind durch die Formel (III) definiert. In dieser Formel stehen $R^4$, $R^5$ und A für diejenigen Reste, die bei der Definition in Formel (I) angegeben sind.

Die Verbindungen der Formel (III) sind bekannt und/oder können nach bekannten Methoden erhalten werden (vgl. DE-A 23 60 362, DE-A 19 61 176, EP-A 180 115, Chemical Abstracts 1953, 12 302 und Chemical Abstracts 1957, 434).

Das bei dem erfindungsgemäßen Verfahren (a-β) weiterhin als Ausgangsstoff einzusetzende Phosgen ist eine allgemein bekannte Verbindung der organischen Chemie.

Die bei dem erfindungsgemäßen Verfahren (a-β) außerdem als Ausgangsstoffe einzusetzenden Amine sind durch die Formel (VI) definiert. In dieser Formel steht $R^4$, $R^5$ und A für diejenigen Reste, die bei der Definition in Formel (I) angegeben sind.

Die Verbindungen der Formel (VI) sind allgemein bekannte Verbindungen der organischen Chemie.

Die bei dem erfindungsgemäßen Verfahren (b) als Ausgangsstoffe einzusetzenden Pyrimidin-4-yl-caramidsäureester sind durch die Formel (Ia) definiert. In dieser Formel stehen $R^{1-1}$, $R^2$, $R^3$, $R^4$, $R^5$ und A

für diejenigen Reste, die bei der Definition in Formel (I) angegeben sind.

Die Verbindungen der Formel (Ia) sind neu und Gegenstand der Erfindung. Sie können nach Verfahren (a-$\alpha$) und (a-$\beta$) erhalten werden.

Die erfindungsgemäßen Verfahren (a-$\alpha$) und (A-$\beta$) zur Herstellung der neuen Verbindungen der Formel (I) werden vorzugsweise unter Verwendung von Verdünnungsmitteln und Säureakzeptoren durchgeführt.

Als Verdünnungsmittel kommen dabei praktische alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-ketone, Ester wie Essigsäuremethylester und -ethylester, Nitrile wie z.B. Acetonitril und Propionitril, Amide wie z.B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei den erfindungsgemäßen Verfahren alle üblicherweise für derartige Umsetzungen verwendbaren Säurebindemittel eingesetzt werden. Vorzugsweise in Frage kommen Erdalkalihydroxide wie z.B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei den erfindungsgemäßen Verfahren (a-$\alpha$) und (a-$\beta$) in einem größeren Bereich variiert werden. Im allgemeinen arbeitet man bei Temperaturen zwischen 0 °C und 100 °C, vorzugsweise bei Temperaturen zwischen 10 °C und 80 °C.

Die erfindungsgemäßen Verfahren (a-$\alpha$) und (a-$\beta$) werden im allgemeinen unter Normaldruck durchgeführt. Es ist jedoch auch möglich, unter erhöhtem oder vermindertem Druck zu arbeiten.

Zur Durchführung der erfindungsgemäßen Verfahren (a-$\alpha$) und (a-$\beta$) setzt man die Ausgangsstoffe im allgemeinen in angenähert äquimolaren Mengen ein. Ein Überschuß der einen oder anderen Komponente bis etwa 10 % ist jedoch problemlos möglich.

Die Reaktionskomponenten werden im allgemeinen in einem der oben angegebenen Lösungsmittel zusammengegeben und mehrere Stunden gerührt, wobei die Temperatur im oben angegebenen Rahmen gehalten wird.

Zu der nach üblichen Methoden erfolgenden Aufarbeitung wird eingeengt, der Rückstand mit Wasser versetzt und das auskristallisierte Produkt abgesaugt, gewaschen und getrocknet.

Das erfindungsgemäße Verfahren (b) wird vorzugsweise in Gegenwart von Wasser als Verdünnungsmittel und in Gegenwart von katalytischen Mengen Molybdänsalzen wie z.B. Ammoniummolybdat durchgeführt.

Das erfindungsgemäße Verfahren (b) wird im allgemeinen bei Temperaturen zwischen -20 °C und +100 °C durchgeführt. Bevorzugt ist der Temperaturbereich zwischen 0 °C und 80 °C, insbesondere zwischen 20 °C und 60 °C. Die Umsetzungen werden im allgemeinen bei Normaldruck durchgeführt.

Zur Durchführung des erfindungsgemäßen Verfahrens (b) wird eine Mischung aus 1 Mol (2-Alkylthioalkylen-pyrimidin-4-yl)-carbamidsäureester (Ia), 0,01 bis 10,0 g, vorzugsweise 0,05 bis 5,0 g, insbesondere 0,1 bis 2,0 g Ammoniummolybdat und 10 bis 500 ml, vorzugsweise 20 bis 300 ml, insbesondere 50 bis 200 ml Wasser tropfenweise und gegebenenfalls unter Kühlung versetzt mit 1 bis 3,5 Mol, vorzugsweise 1,5 bis 3,0 Mol, insbesondere 2,0 bis 2,8 Mol Oxidationsmittel (z.B. Wasserstoffperoxid). Nach Ablauf der Reaktion wird abgesaugt, gewaschen und getrocknet.

Die Verbindungen der Formel (I) fallen in der Regel in fester Form an und können durch Umkristallisation gereinigt werden. Zur Charakterisierung dient der Schmelzpunkt.

Gegenstand der Erfindung sind außer den Stoffen der obigen Formel (I) und den oben beschriebenen Verfahren zu deren Herstellung auch

- Schädlingsbekämpfungsmittel, insbesondere insektizide Mittel, gekennzeichnet durch einen Gehalt an mindestens einen Pyrimidin-4-yl-carbamidsäureester der Formel (I);
- Verfahren zur Bekämpfung von tierischen Schädlingen, insbesondere von Insekten, dadurch gekennzeichnet, daß man Pyrimidin-4-yl-carbamidsäureester der Formel (I) auf tierische Schädlinge und/oder deren Lebensraum einwirken läßt, bzw. die Verwendung von Pyridin-4-yl-carbamidsäureestern der Formel (I) zur Bekämpfung von tierischen Schädlingen, insbesondere von Insekten, wobei jedoch die vom Gesetz her nicht schutzfähigen Verwendungen ausgenommen sind; sowie
- Verfahren zur Herstellung von Mitteln gegen tierische Schädlinge, dadurch gekennzeichnet, daß man Pyrimidin-4-yl-carbamidsäureester der Formel (I) mit Streckmitteln und/oder oberflächenaktiven Mitteln vermischt.

Hierzu werden nachfolgend nähere Erläuterungen gegeben:

Die Wirkstoffe eignen sich zur Bekämpfung von tierischen Schädlingen, vorzugsweise Arthropoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola biseelliella, Tinea pellionella, Hofmannophila pseudospretella, Cacoecia podana, Capua reticulans, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe der Formel (I) zeichnen sich durch eine hervorragende insektizide Wirksamkeit aus. Sie zeigen insbesondere beim Einsatz als Bodeninsektizide eine hervorragende Wirkung gegen Blattläuse wie z.B. Myzus persicae und Aphis fabae.
Die Verbindungen der Formel (I) zeichnen sich außerdem durch eine besonders gute systemische und wurzelsystemische Wirksamkeit aus.

Die neuen Verbindungen sind also besonders gut für einen Einsatz zur langanhaltenden Bekämpfung von Bodeninsekten geeignet.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die Herstellung und die Verwendung der erfindungsgemäßen Wirkstoffe geht aus den folgenden Beispielen hervor.

Herstellungsbeispiele:

Beispiel 1:

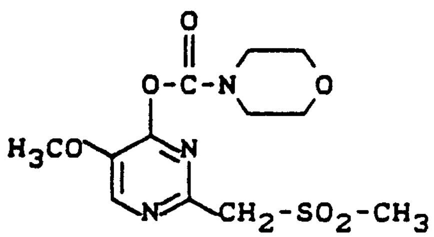

(Verfahren a-$\alpha$)

Zu einer Mischung aus 21,8 g (0,1 Mol) 2-Methylsulfonyl-4-hydroxy-5-methoxypyrimidin, 16,6 g (0,12 Mol) Kaliumcarbonat, 0,6 g Diazabicyclooctan und 200 ml Aceton gibt man 15 g (0,1 Mol) Morpholinocarbamidsäurechlorid. Das Reaktionsgemisch wird 18 Stunden bei 40°C gerührt, dann destilliert man das Lösungsmittel unter vermindertem Druck ab und verrührt den Rückstand mit 100 ml Wasser. Man saugt das ausgefallene Produkt ab und erhält so 12,5 g (38 % der Theorie) O-(2-Methylsulfonylmethyl-5-methoxypyrimidin-4-yl)-morpholino-carbamidsäureester in Form weißer Kristalle mit dem Schmelzpunkt 138°C.

Beispiel 2:

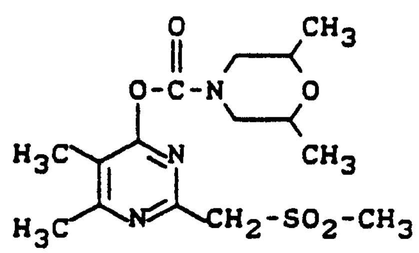

Verfahren (a-$\beta$)

Zu 30 g einer 20 %igen Phosgenlösung in Toluol tropft man bei -8°C bis -3°C eine Lösung von 11 g (0,06 Mol) 2-Methylsulfonylmethyl-4-hydroxy-5,6-dimethylpyrimidin und 8,6 ml (0,062 Mol) Triethylamin in 200 ml Acetonitril. Das Reaktionsgemisch wird 1 Stunde bei -5°C bis 0°C nachgerührt und dann bei 0 bis 10°C mit einer Mischung aus 6,9 g (0,06 Mol) 2,6-Dimethylmorpholin und 9,6 g (0,07 Mol) Triethylamin versetzt. Man rührt die Reaktionslösung 18 Stunden bei Raumtemperatur nach, dampft das Lösungsmittel unter vermindertem Druck ab, gibt 200 ml Wasser zu und extrahiert zweimal mit je 150 ml Methylenchlorid. Die organischen Phasen werden über Natriumsulfat getrocknet und unter vermindertem Druck eingedampft. Den Rückstand verreibt man mit 50 ml Petrolether und saugt das Produkt nach Kristallisation ab.
Man erhält so 14,3 g (67 % der Theorie) 2,6-Dimethylmorpholinocarbamidsäure-O-(2-methylthiomethyl-5,6-dimethylpyrimidin-4-yl)-ester in Form farbloser Kristalle mit dem Schmelzpunkt 103°C.

Beispiel 3:

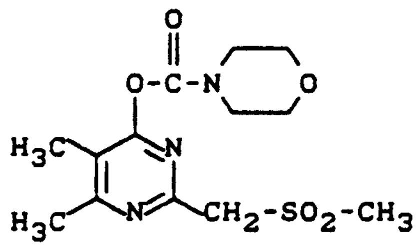

Verfahren (b)

Eine Mischung aus 8,9 g (0,03 Mol) O-(2-Methylthiomethyl-5,6-dimethyl-pyrimidin-4-yl)-morpholinocarbamidsäureester, 50 ml Wasser und 0,1 g Ammoniummolybdat wird mit 8,5 g (0,075 Mol) 30 %-igem Wasserstoffperoxid versetzt und 18 Stunden lang gerührt. Dann saugt man das ausgefallene Produkt ab, wäscht mit 50 ml Eiswasser nach und trocknet an der Luft.

Man erhält so 6,3 g (64 % der Theorie) O-(2-Methylsulfonylmethyl-5,6-dimethyl-pyrimidin-4-yl)-morpholinocarbamidsäureester in Form farbloser Kristalle mit dem Schmelzpunkt 147 °C.

In analoger Weise zu den Beispielem 1 bis 3 und unter Berücksichtigung der Angaben in der Beschreibung zu den erfindungsgemäßen Verfahren, werden die nachstehend in Tabelle 2 aufgeführten Endprodukte der Formel (I)

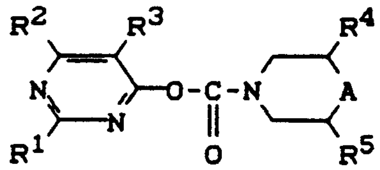

(I)

erhalten:

**Tabelle 2:**

| Bsp. Nr. | $R^1$ | $R^2$ | $R^3$ | $R^4$ | $R^5$ | A | Schmp. [°C]/ Brechungsindex |
|---|---|---|---|---|---|---|---|
| 4 | $CH_2SO_2CH_3$ | $-CH_2CH_2CH_2-$ | | H | H | O | 144 |
| 5 | $CH_2SCH_3$ | H | $OCH_3$ | H | H | O | 51 |
| 5 | $CH_2SCH_3$ | $CH_3$ | $CH_3$ | H | H | O | 54 |
| 7 | $N(CH_3)_2$ | $CH_3$ | $CH_3$ | H | H | O | 82 |
| 8 | $CH_2SCH_3$ | $-CH_2CH_2CH_2-$ | | H | H | O | 108 |
| 9 | $CH_2SO_2CH_3$ | $CH_3$ | $CH_3$ | H | H | S | 138 |
| 10 | $CH_2SO_2CH_3$ | $CH_3$ | $CH_3$ | H | H | $N-CH_3$ | 101 |
| 11 | $CH_2SO_2CH_3$ | H | $CH_3$ | H | H | O | 122 |
| 12 | $CH_2CH_2SCH_3$ | H | $CH_3$ | H | H | O | $n_D^{20}$ 1,5462 |
| 13 | $CH_2CH_2SO_2-CH_3$ | H | $CH_3$ | H | H | O | 106 |
| 14 | $N(CH_3)_2$ | $-CH_2CH_2CH_2-$ | | H | H | O | 104 |
| 15 | $CH_2SCH_3$ | H | $C_3H_7-i$ | H | H | O | $n_D^{20}$ : 1,5441 |
| 16 | $CH_2SO_2CH_3$ | H | $C_3H_7-i$ | H | H | O | 118 |

Verwendungsbeispiele

In den folgenden Verwendungsbeispielen wurde die folgende Verbindung als Vergleichssubstanz eingesetzt:

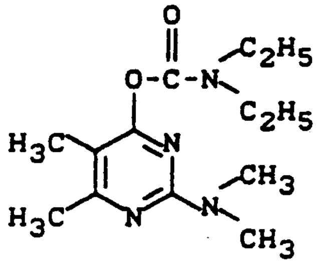

(A)

2-Dimethylamino-5,6-dimethylpyrimidin-4-yl-dimethylcarbamat
(bekannt aus GB-A 1181 657)

Beispiel A

Myzus-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

In diesem Test zeigten z.B. die folgenden Verbindungen der Herstellungsbeispiele (1), (3), (4), (5), (6), (7) und (8) nach 1 Tag einen Abtötungsgrad von 85 bis 100 % gegenüber 10 % der Vergleichsverbindung (A).

## Tabelle A

(pflanzenschädigende Insekten)
Myzus - Test

| Wirkstoff | Wirkstoffkonzentration in % | Abtötungsgrad in % nach 1 d |
|---|---|---|
| 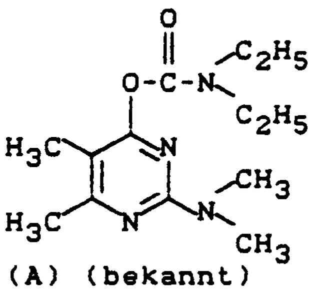 (A) (bekannt) | 0,01 | 10 |
| 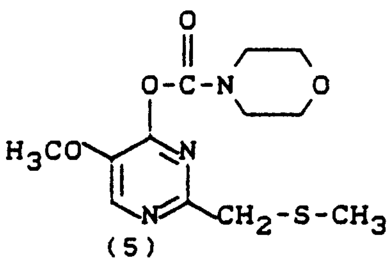 (5) | 0,01 | 80 |
| 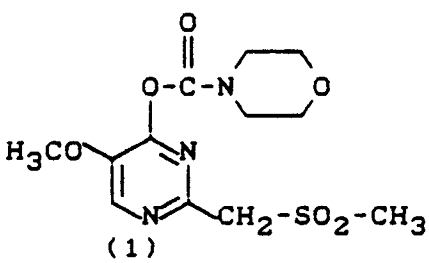 (1) | 0,01 | 90 |
| 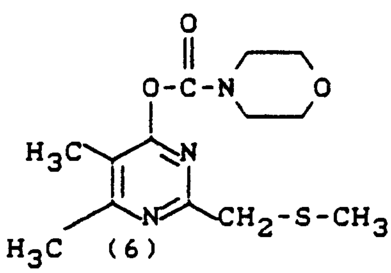 (6) | 0,01 | 85 |

## Tabelle A - Fortsetzung

(pflanzenschädigende Insekten)
Myzus - Test

| Wirkstoff | Wirkstoffkonzentration in % | Abtötungsgrad in % nach 1 d |
|---|---|---|
| 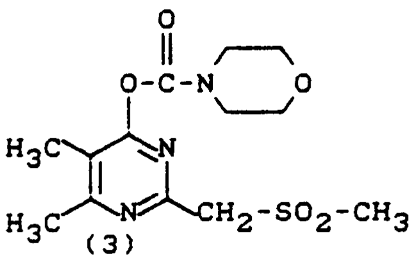 (3) | 0,01 | 98 |
| 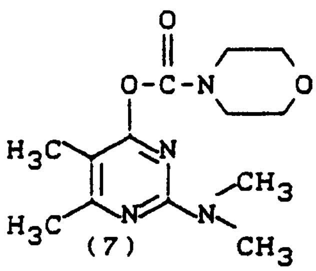 (7) | 0,01 | 100 |
| 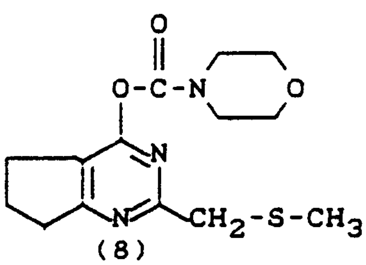 (8) | 0,01 | 100 |
| 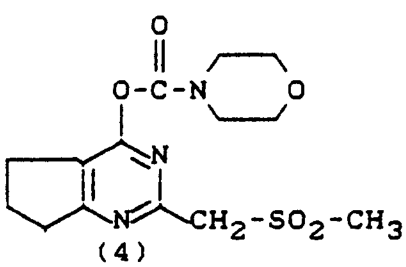 (4) | 0,01 | 98 |

Beispiel B

Aphis-Test (systemische Wirkung)

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat

mit Wasser auf die gewünschte Konzentration.

Mit je 20 ml Wirkstoffzubereitung der gewünschten Konzentration werden Bohnenpflanzen (Vicia faba), die stark von der schwarzen Bohnenlaus (Aphis fabae) befallen sind, angegossen, so daß die Wirkstoffzubereitung in den Boden eindringt, ohne den Sproß zu benetzen. Der Wirkstoff wird von den Wurzeln aufgenommen und in den Sproß weitergeleitet.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

In diesem Test zeigten z.B. die folgenden Verbindungen der Herstellungsbeispiele (3), (4), (5), (6), (7) und (8) nach 4 Tag einen Abtötungsgrad von 95 bis 100 % gegenüber 20 % der Vergleichsverbindung (A).

## Tabelle B

(pflanzenschädigende Insekten)
Aphis-Test (systemisch)

| Wirkstoff | Wirkstoffkonzentration in % | Abtötungsgrad in % nach 4 d |
|---|---|---|
| 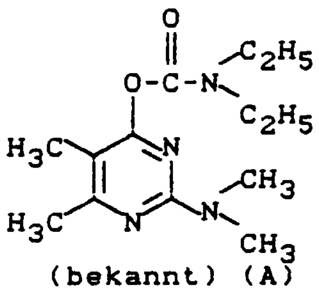 (bekannt) (A) | 0,01 | 20 |
| 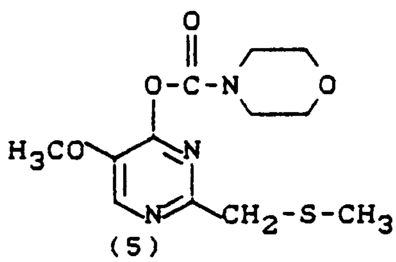 (5) | 0,01 | 95 |
| 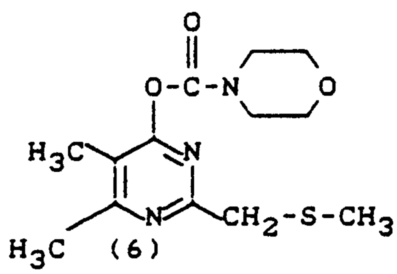 (6) | 0,01 | 95 |
| 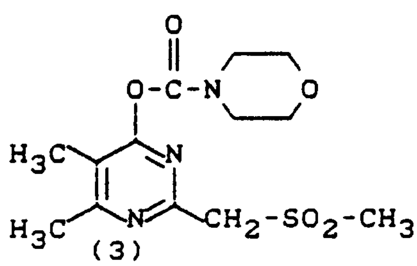 (3) | 0,01 | 100 |

Tabelle B - Fortsetzung

(pflanzenschädigende Insekten)
Aphis-Test (systemisch)

| Wirkstoff | Wirkstoffkonzentration in % | Abtötungsgrad in % nach 4 d |
|---|---|---|
| 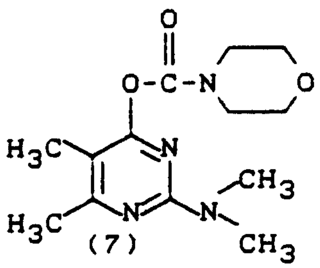 (7) | 0,01 | 98 |
| 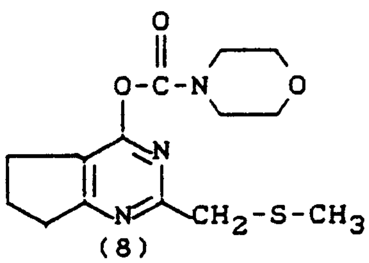 (8) | 0,01 | 98 |
| 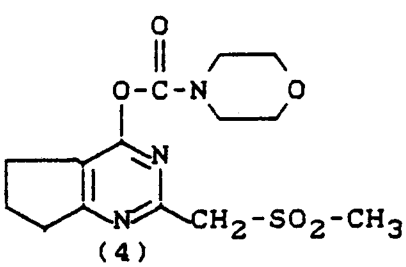 (4) | 0,01 | 100 |

Beispiel C

Grenzkonzentrations-Test / Wurzelsystemische Wirkung

Testinsekt: Myzus persicae
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzübereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

In diesem Test zeigten z.B. die folgenden Verbindungen der Herstellungsbeispiele (1), (3), (4), (5), (6), (7) und (8) bei einer beispielhaften Wirkstoffkonzentration von 10 ppm eine Abtötung von 100 %.

**Tabelle C**

Wurzelsystemische Wirkung
*Myzus persicae*

| Wirkstoff | Abtötungsgrad in % bei Wirkstoffkonzentration in ppm |
|---|---|
| 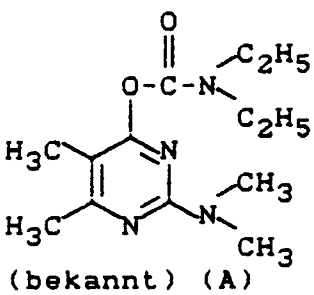 (bekannt) (A) | 10 ppm = 0 % |
| 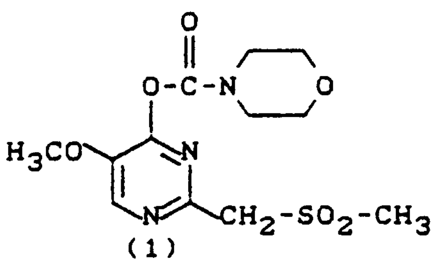 (1) | 10 ppm = 100 % |
| 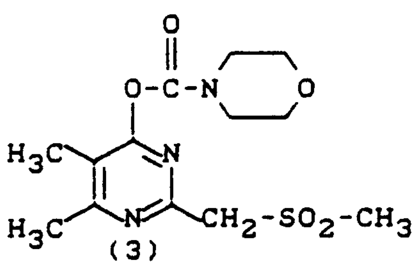 (3) | 10 ppm = 100 % |
| 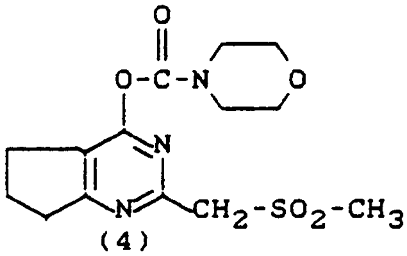 (4) | 10 ppm = 100 % |

## Tabelle C - Fortsetzung

Wurzelsystemische Wirkung
Myzus persicae

| Wirkstoff | Abtötungsgrad in % bei Wirkstoffkonzentration in ppm |
|---|---|
| 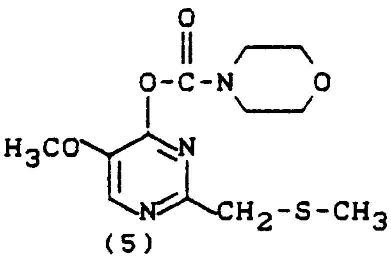 (5) | 10 ppm = 100 % |
| 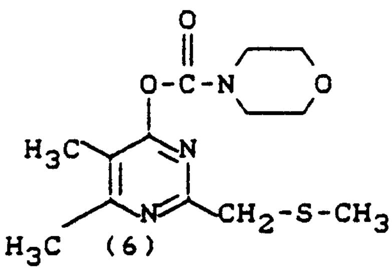 (6) | 10 ppm = 100 % |
| 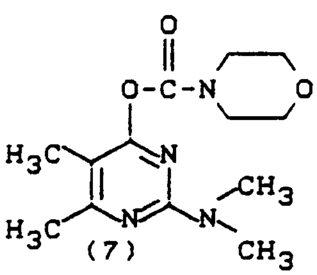 (7) | 10 ppm = 100 % |
| 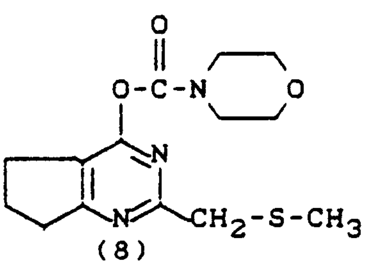 (8) | 10 ppm = 100 % |

Beispiel D

Grenzkonzentrations-Test / Wurzelsystemische Wirkung

| | |
|---|---|
| Testinsekt: | Myzus persicae |
| Lösungsmittel: | 3 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachteil des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele (11), (12), (13), (15) und (16) bei einer beispielhaften Wirkstoffkonzentration von 10 ppm eine Abtötung von 100 %.

## Tabelle D

Wurzelsystemische Wirkung
Myzus persicae

| Wirkstoff (Konstitution) | Abtötungsgrad in % bei Wirkstoffkonzentration in ppm |
|---|---|
| 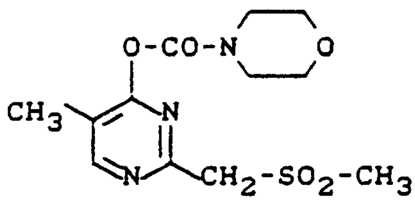 (11) | 10 ppm = 100 % |
| 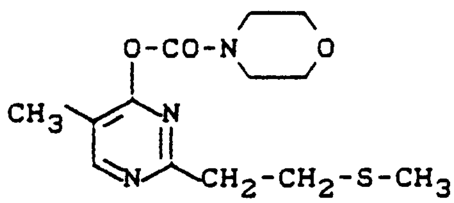 (12) | 10 ppm = 100 % |
| 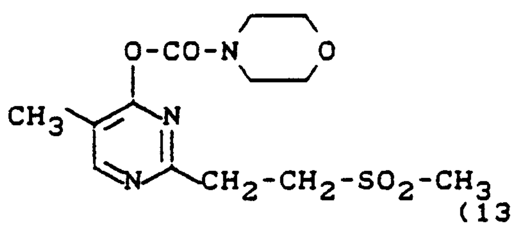 (13) | 10 ppm = 100 % |
| 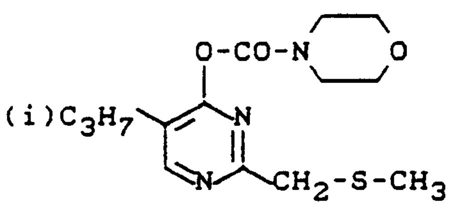 (15) | 10 ppm = 100 % |
| 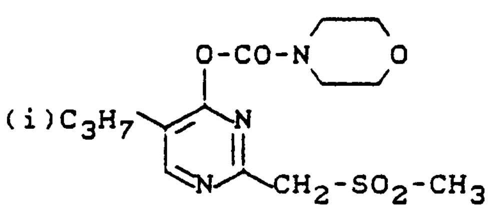 (16) | 10 ppm = 100 % |

## Patentansprüche

## Patentansprüche für folgende Vertragsstaaten : BE, CH, DE, DK, FR, GB, IT, LI, NL

**1.** Pyrimidin-4-yl-carbamidsäureester der Formel (I)

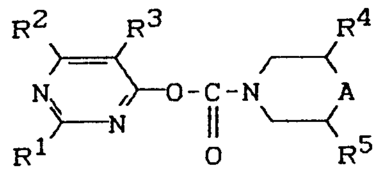

(I)

in welcher

$R^1$ für Alkoxy mit 1 bis 4 Kohlenstoffatomen, Alkylthio mit 1 bis 4 Kohlenstoffatomen, Alkylthioalkyl mit 1 bis 4 Kohlenstoffatomen in den einzelnen Alkylteilen, Alkylsulfinylalkyl mit 1 bis 4 Kohlenstoffatomen in den einzelnen Alkylteilen, Alkylsulfonylalkyl mit 1 bis 4 Kohlenstoffatomen in den einzelnen Alkylteilen und Dialkylamino mit 1 bis 3 Kohlenstoffatomen in den einzelnen Alkylteilen steht,

$R^2$ für Wasserstoff oder Alkyl mit 1 bis 4 Kohlenstoffatomen steht,

$R^3$ für Wasserstoff, Alkyl mit 1 bis 4 Kohlenstoffatomen oder Alkoxy mit 1 bis 4 Kohlenstoffatomen steht oder

$R^2$ und $R^3$ gemeinsam für einen zweifach verknüpften, geradkettigen oder verzweigten Alkandiylrest mit 2 bis 5 Kohlenstoffatomen stehen,

$R^4$ für Wasserstoff oder Alkyl mit 1 bis 3 Kohlenstoffatomen steht,

$R^5$ für Wasserstoff oder Alkyl mit 1 bis 3 Kohlenstoffatomen steht und

A für Sauerstoff, Schwefel oder für einen Rest

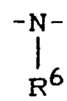

steht, wobei

$R^6$ für Alkyl mit 1 bis 3 Kohlenstoffatomen steht.

**2.** Pyrimidin-4-yl-carbamidsäureester der Formel (I) gemäß Anspruch 1,
in welcher

$R^1$ für Methoxy, Ethoxy, Methylthio, Ethylthio, Methylthiomethyl, Ethylthiomethyl, 2-Methylthioethyl, Methylsulfinylmethyl, Ethylsulfinylmethyl, 2-Methylsulfinylethyl, Methylsulfonylmethyl, Ethylsulfonylmethyl, 2-Methylsulfonylethyl, Dimethylamino oder Diethylamino steht,

$R^2$ für Wasserstoff, Methyl oder Ethyl steht,

$R^3$ für Wasserstoff, Methyl, Ethyl, Isopropyl, Methoxy oder Ethoxy steht oder

$R^2$ und $R^3$ gemeinsam für einen zweifach verknüpften Alkandiylrest mit 3 oder 4 Kohlenstoffatomen stehen,

$R^4$ für Wasserstoff oder Methyl steht,

$R^5$ für Wasserstoff oder Methyl steht und

A für Sauerstoff, Schwefel oder für den Rest

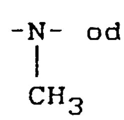 oder 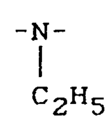

steht.

3. Verfahren zur Herstellung von Pyrimidin-4-yl-carbamidsäureestern der Formel (I) gemäß Anspruch 1, dadurch gekennzeichnet, daß man entweder

a) 4-Hydroxy-pyrimidin-Derivate der Formel (II)

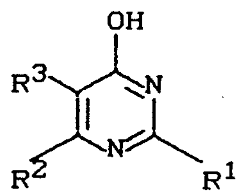

(II)

in welcher

$R^1$, $R^2$ und $R^3$ die in Anspruch 1 angegebene Bedeutung haben,

oder das entsprechende Alkalimetall-, Erdalkalimetall- oder Ammoniumsalz

($\alpha$) mit Carbamidsäurechloriden der Formel (III)

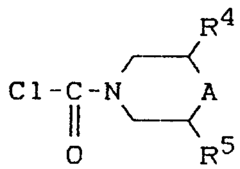

(III)

in welcher

$R^4$, $R^5$ und A die in Anspruch 1 angegebene Bedeutung haben,

gegebenenfalls in Gegenwart eines Säureakzeptors und gegebenenfalls in Gegenwart eines Verdünnungsmittels umsetzt oder

($\beta$) mit Phosgen der Formel (IV)

$COCl_2$ (IV)

gegebenenfalls in Gegenwart eines Säureakzeptors und gegebenenfalls in Gegenwart eines Verdünnungsmittels umsetzt

und die sich bildenden Chlorameisensäure-pyrimidylester der Formel (V)

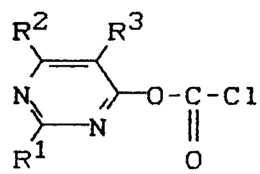

(V)

in welcher

$R^1$, $R^2$ und $R^3$ die in Anspruch 1 angegebene Bedeutung haben,

gegebenenfalls nach der Isolierung mit Aminen der allgemeinen Formel (VI)

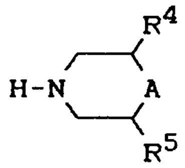

(VI)

in welcher

$R^4$, $R^5$ und A die in Anspruch 1 angegebene Bedeutung haben,

gegebenenfalls in Gegenwart eines Säureakzeptors und gegebenenfalls in Gegenwart eines Verdünnungsmittels umsetzt

oder

b) die nach Verfahren (a-α) und (a-β) erhältlichen Pyrimidin-4-yl-carbamidsäureester der Formel (Ia)

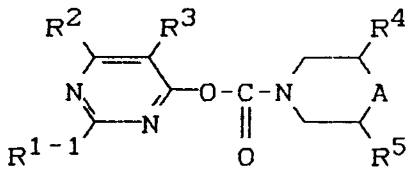

(Ia)

in welcher

$R^2$, $R^3$, $R^4$, $R^5$ und A die in Anspruch 1 angegebene Bedeutung haben und

$R^{1-1}$ für Alkylthioalkyl mit 1 bis 4 Kohlenstoffatomen in den einzelnen Alkylteilen steht,

mit 1 bis 3,5 Mol eines Oxidationsmittels pro Mol Carbamidsäureester (Ia), gegebenenfalls in Gegenwart von Verdünnungsmitteln und gegebenenfalls in Gegenwart eines Katalysators, umsetzt und die Verbindungen der allgemeinen Formel (I) isoliert.

**4.** Schädlingsbekämpfungsmittel, gekennzeichnet durch einen Gehalt an mindestens einem Pyrimidin-4-yl-carbamidsäureester der Formel (I) gemäß Anspruch 1.

**5.** Insektizide Mittel, gekennzeichnet durch einen Gehalt an mindestens einem Pyrimidin-4-yl-carbamidsäureester der Formel (I) gemäß Anspruch 1.

**6.** Verfahren zur Bekämpfung von tierischen Schädlingen, insbesondere von Insekten, dadurch gekennzeichnet, daß man Pyrimidin-4-yl-carbamidsäureester der Formel (I) gemäß Anspruch 1 auf tierische Schädlinge und/oder deren Lebensraum einwirken läßt.

**7.** Verwendung von Pyrimidin-4-yl-carbamidsäureestern der Formel (I) gemaß Anspruch 1 zur Bekämpfung von tierischen Schädlingen, insbesondere von Insekten, ausgenommen die vom Art.52(4) EPÜ her nicht schutzfähigen Verwendungen.

**8.** Verfahren zur Herstellung von Mitteln gegen tierische Schädlinge, dadurch gekennzeichnet, daß man Pyrimidin-4-yl-carbamidsäureester der Formel (I) gemäß Anspruch 1 mit Streckmitteln und/oder oberflächenaktiven Mitteln vermischt.

**Patentansprüche für folgenden Vertragsstaat : ES**

1. Verfahren zur Herstellung von Pyrimidin-4-yl-carbamidsäureestern der Formel (I)

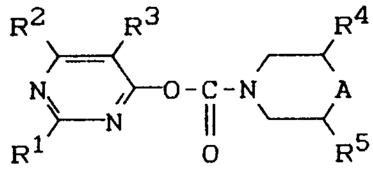

(I)

in welcher

| | |
|---|---|
| $R^1$ | für Alkoxy mit 1 bis 4 Kohlenstoffatomen, Alkylthio mit 1 bis 4 Kohlenstoffatomen, Alkylthioalkyl mit 1 bis 4 Kohlenstoffatomen in den einzelnen Alkylteilen, Alkylsulfinylalkyl mit 1 bis 4 Kohlenstoffatomen in den einzelnen Alkylteilen, Alkylsulfonylalkyl mit 1 bis 4 Kohlenstoffatomen in den einzelnen Alkylteilen und Dialkylamino mit 1 bis 3 Kohlenstoffatomen in den einzelnen Alkylteilen steht, |
| $R^2$ | für Wasserstoff oder Alkyl mit 1 bis 4 Kohlenstoffatomen steht, |
| $R^3$ | für Wasserstoff, Alkyl mit 1 bis 4 Kohlenstoffatomen oder Alkoxy mit 1 bis 4 Kohlenstoffatomen steht oder |
| $R^2$ und $R^3$ | gemeinsam für einen zweifach verknüpften, geradkettigen oder verzweigten Alkandiylrest mit 2 bis 5 Kohlenstoffatomen stehen, |
| $R^4$ | für Wasserstoff oder Alkyl mit 1 bis 3 Kohlenstoffatomen steht, |
| $R^5$ | für Wasserstoff oder Alkyl mit 1 bis 3 Kohlenstoffatomen steht und |
| A | für Sauerstoff, Schwefel oder für einen Rest |

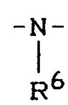

steht, wobei

| | |
|---|---|
| $R^6$ | für Alkyl mit 1 bis 3 Kohlenstoffatomen steht, |

dadurch gekennzeichnet, daß man entweder

a) 4-Hydroxy-pyrimidin-Derivate der Formel (II)

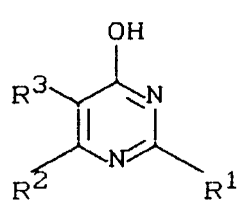

(II)

in welcher

| | |
|---|---|
| $R^1$, $R^2$ und $R^3$ | die oben angegebene Bedeutung haben, |
| | oder das entsprechende Alkalimetall-, Erdalkalimetall- oder Ammoniumsalz |

($\alpha$) mit Carbamidsäurechloriden der Formel (III)

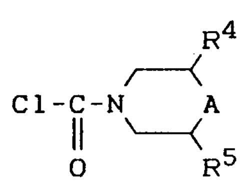

(III)

in welcher

$R^4$, $R^5$ und A die oben angegebene Bedeutung haben,

gegebenenfalls in Gegenwart eines Säureakzeptors und gegebenenfalls in Gegenwart eines Verdünnungsmittels umsetzt oder

($\beta$) mit Phosgen der Formel (IV)

$COCl_2$ (IV)

gegebenenfalls in Gegenwart eines Säureakzeptors und gegebenenfalls in Gegenwart eines Verdünnungsmittels umsetzt

und die sich bildenden Chlorameisensäure-pyrimidylester der Formel (V)

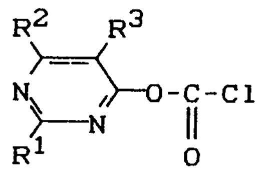

(V)

in welcher

$R^1$, $R^2$ und $R^3$ die oben angegebene Bedeutung haben,

gegebenenfalls nach der Isolierung mit Aminen der allgemeinen Formel (VI)

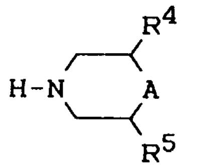

(VI)

in welcher

$R^4$, $R^5$ und A die oben angegebene Bedeutung haben,

gegebenenfalls in Gegenwart eines Säureakzeptors und gegebenenfalls in Gegenwart eines Verdünnungsmittels umsetzt

oder

b) die nach Verfahren (a-$\alpha$) und (a-$\beta$) erhältlichen Pyrimidin-4-yl-carbamidsäureester der Formel (Ia)

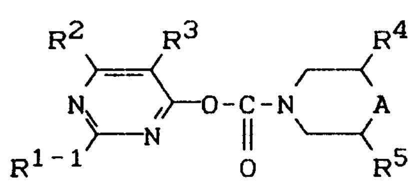

(Ia)

in welcher

$R^2$, $R^3$, $R^4$, $R^5$ und A die oben angegebene Bedeutung haben und

$R^{1-1}$ für Alkylthioalkyl mit 1 bis 4 Kohlenstoffatomen in den einzelnen Alkylteilen steht,

mit 1 bis 3,5 Mol eines Oxidationsmittels pro Mol Carbamidsäureester (Ia), gegebenenfalls in Gegenwart von Verdünnungsmitteln und gegebenenfalls in Gegenwart eines Katalysators, umsetzt und die Verbindungen der allgemeinen Formel (I) isoliert.

**2.** Verfahren zur Herstellung von Pyrimidin-4-yl-carbamidsäureestern der Formel (I) gemäß Anspruch 1, dadurch gekennzeichnet, daß in den Formeln (I), (Ia), (II), (III), (IV), (V) bzw. (VI) jeweils

$R^1$ für Methoxy, Ethoxy, Methylthio, Ethylthio, Methylthiomethyl, Ethylthiomethyl, 2-Me-

| | |
|---|---|
| | thylthioethyl, Methylsulfinylmethyl, Ethylsulfinylmethyl, 2-Methylsulfinylethyl, Methylsulfonylmethyl, Ethylsulfonylmethyl, 2-Methylsulfonylethyl, Dimethylamino oder Diethylamino steht, |
| $R^2$ | für Wasserstoff, Methyl oder Ethyl steht, |
| $R^3$ | für Wasserstoff, Methyl, Ethyl, Isopropyl, Methoxy oder Ethoxy steht oder |
| $R^2$ und $R^3$ | gemeinsam für einen zweifach verknüpften Alkandiylrest mit 3 oder 4 Kohlenstoffatomen stehen, |
| $R^4$ | für Wasserstoff oder Methyl steht, |
| $R^5$ | für Wasserstoff oder Methyl steht und |
| A | für Sauerstoff, Schwefel oder für den Rest |

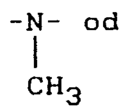 oder 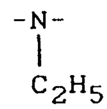

steht.

3. Schädlingsbekämpfungsmittel, gekennzeichnet durch einen Gehalt an mindestens einem Pyrimidin-4-yl-carbamidsäureester der Formel (I) gemäß Anspruch 1.

4. Insektizide Mittel, gekennzeichnet durch einen Gehalt an mindestens einem Pyrimidin-4-yl-carbamidsäureester der Formel (I) gemäß Anspruch 1.

5. Verfahren zur Bekämpfung von tierischen Schädlingen, insbesondere von Insekten, dadurch gekennzeichnet, daß man Pyrimidin-4-yl-carbamidsäureester der Formel (I) gemäß Anspruch 1 auf tierische Schädlinge und/oder deren Lebensraum einwirken läßt.

6. Verwendung von Pyrimidin-4-yl-carbamidsäureestern der Formel (I) gemäß Anspruch 1 zur Bekämpfung von tierischen Schädlingen, insbesondere von Insekten, ausgenommen die vom Art.52(4) EPÜ her nicht schutzfähigen Verwendungen.

7. Verfahren zur Herstellung von Mitteln gegen tierische Schädlinge, dadurch gekennzeichnet, daß man Pyrimidin-4-yl-carbamidsäureester der Formel (I) gemäß Anspruch 1 mit Streckmitteln und/oder oberflächenaktiven Mitteln vermischt.

**Claims**

**Claims for the following Contracting States : BE, CH, DE, DK, FR, GB, IT, LI, NL**

1. Pyrimidin-4-yl carbamates of the formula (I)

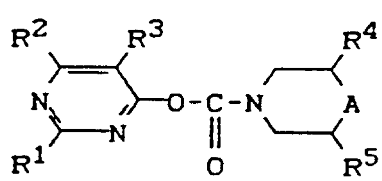 (I)

in which

| | |
|---|---|
| $R^1$ | represents alkoxy having 1 to 4 carbon atoms, alkylthio having 1 to 4 carbon atoms, alkylthioalkyl having 1 to 4 carbon atoms in the individual alkyl moieties, alkylsulphinylalkyl having 1 to 4 carbon atoms in the individual alkyl moieties, alkylsulphonylalkyl having 1 to 4 carbon atoms in the individual alkyl moieties and dialkylamino having 1 to 3 carbon atoms in the individual alkyl moieties, |
| $R^2$ | represents hydrogen or alkyl having 1 to 4 carbon atoms, |
| $R^3$ | represents hydrogen, alkyl having 1 to 4 carbon atoms or alkoxy having 1 to 4 carbon |

| | |
|---|---|
| | atoms or |
| $R^2$ and $R^3$ | together represent a double-linked, straight-chain or branched alkanediyl radical having 2 to 5 carbon atoms, |
| $R^4$ | represents hydrogen or alkyl having 1 to 3 carbon atoms, |
| $R^5$ | represents hydrogen or alkyl having 1 to 3 carbon atoms and |
| A | represents oxygen, sulphur or a radical |

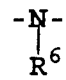

where

| | |
|---|---|
| $R^6$ | represents alkyl having 1 to 3 carbon atoms. |

**2.** Pyrimidin-4-yl carbamates of the formula (I) according to Claim 1, in which

| | |
|---|---|
| $R^1$ | represents methoxy, ethoxy, methylthio, ethylthio, methylthiomethyl, ethylthiomethyl, 2-methylthioethyl,methylsulphinylmethyl, ethylsulphinylmethyl, 2-methylsulphinylethyl, methylsulphonylmethyl, ethylsulphonylmethyl, 2-methylsulphonylethyl, dimethylamino or diethylamino, |
| $R^2$ | represents hydrogen, methyl or ethyl, |
| $R^3$ | represents hydrogen, methyl, ethyl, isopropyl, methoxy or ethoxy or |
| $R^2$ and $R^3$ | together represent a double-linked alkanediyl radical having 3 or 4 carbon atoms, |
| $R^4$ | represents hydrogen or methyl, |
| $R^5$ | represents hydrogen or methyl and |
| A | represents oxygen, sulphur or the radical |

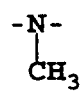

or

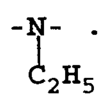 .

**3.** Process for the preparation of pyrimidin-4-yl carbamates of the formula (I) according to Claim 1, characterized in that either

a) 4-hydroxy-pyrimidine derivatives of the formula (II)

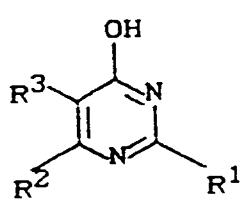 (II)

in which

$R^1$, $R^2$ and $R^3$ have the meaning given in Claim 1,
or the corresponding alkali metal salt, alkaline earth metal salt or ammonium salt,
(α) are reacted with carbamic acid chlorides of the formula (III)

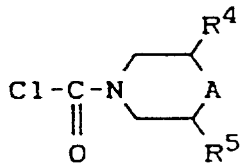

(III)

in which
$R^4$, $R^5$ and A have the meaning given in Claim 1,
if appropriate in the presence of an acid acceptor and, if appropriate, in the presence of a diluent,
or
(β) are reacted with phosgene, of the formula (IV)

$COCl_2$ (IV)

if appropriate in the presence of an acid acceptor and, if appropriate, in the presence of a diluent, and the resulting pyrimidyl chloroformates of the formula (V)

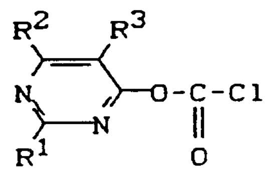

(V)

in which
$R^1$, $R^2$ and $R^3$ have the meaning given in Claim 1,
are isolated, if appropriate, and then reacted with amines of the general formula (VI)

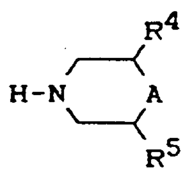

(VI)

in which
$R^4$, $R^5$ and A have the meaning given in Claim 1,
if appropriate in the presence of an acid acceptor and if appropriate in the presence of a diluent,
or
b) the pyrimidin-4-yl carbamates which can be obtained by processes (a-α) and (a-β), of the formula (Ia)

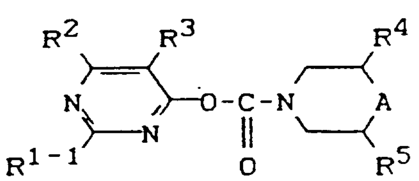

(Ia)

in which

$R^2$, $R^3$, $R^4$, $R^5$ and A have the meaning given in Claim 1 and

$R^{1-1}$ represents alkylthioalkyl having 1 to 4 carbon atoms in the individual alkyl moieties,

are reacted with 1 to 3.5 mol of an oxidant per mole of carbamate (Ia), if appropriate in the presence of diluents and, if appropriate, in the presence of a catalyst, and the compounds of the general formula (I) are isolated.

**4.** Pesticides, characterized in that they contain at least one pyrimidin-4-yl carbamate of the formula (I) according to Claim 1.

**5.** Insecticidal agents, characterized in that they contain at least one pyrimidin-4-yl carbamate of the formula (I) according to Claim 1.

**6.** Method of controlling animal pests, in particular insects, characterized in that pyrimidin-4-yl carbamates of the formula (I) according to Claim 1 are allowed to act on animal pests and/or their environment.

**7.** Use of pyrimidin-4-yl carbamates of the formula (I) according to Claim 1 for controlling animal pests, in particular insects, with the exception of the uses which cannot be protected in accordance with Art. 52-(4) EPC.

**8.** Process for the preparation of agents against animal pests, characterized in that pyrimidin-4-yl carbamates of the formula (I) according to Claim 1 are mixed with extenders and/or surface-active agents.

**Claims for the following Contracting State : ES**

**1.** Process for the preparation of pyrimidin-4-yl carbamates of the formula (I)

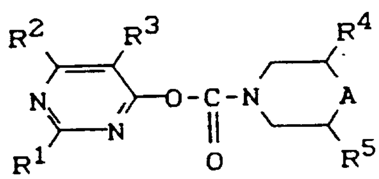

(I)

in which

$R^1$ represents alkoxy having 1 to 4 carbon atoms, alkylthio having 1 to 4 carbon atoms, alkylthioalkyl having 1 to 4 carbon atoms in the individual alkyl moieties, alkylsulphinylalkyl having 1 to 4 carbon atoms in the individual alkyl moieties, alkylsulphonylalkyl having 1 to 4 carbon atoms in the individual alkyl moieties and dialkylamino having 1 to 3 carbon atoms in the individual alkyl moieties,

$R^2$ represents hydrogen or alkyl having 1 to 4 carbon atoms,

$R^3$ represents hydrogen, alkyl having 1 to 4 carbon atoms or alkoxy having 1 to 4 carbon atoms or

$R^2$ and $R^3$ together represent a double-linked, straight-chain or branched alkanediyl radical having 2 to 5 carbon atoms,

$R^4$ represents hydrogen or alkyl having 1 to 3 carbon atoms,

$R^5$ represents hydrogen or alkyl having 1 to 3 carbon atoms and

A represents oxygen, sulphur or a radical

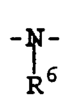

where

$R^6$ represents alkyl having 1 to 3 carbon atoms,

characterized in that either

a) 4-hydroxy-pyrimidine derivatives of the formula (II)

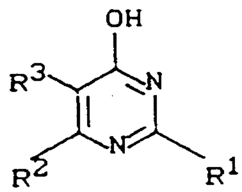

(II)

in which

$R^1$, $R^2$ and $R^3$ have the abovementioned meaning,

or the corresponding alkali metal salt, alkaline earth metal salt or ammonium salt,

(α) are reacted with carbamic acid chlorides of the formula (III)

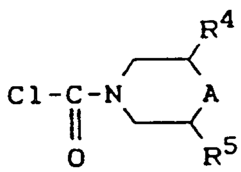

(III)

$R^4$, $R^5$ and A have the abovementioned meaning,

if appropriate in the presence of an acid acceptor and, if appropriate, in the presence of a diluent, or

(β) are reacted with phosgene, of the formula (IV)

$COCl_2$ (IV)

if appropriate in the presence of an acid acceptor and, if appropriate, in the presence of a diluent, and the resulting pyrimidyl chloroformates of the formula (V)

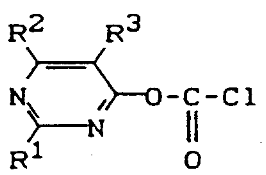

(V)

in which

$R^1$, $R^2$ and $R^3$ have the abovementioned meaning,

are isolated, if appropriate, and then reacted with amines of the general formula (VI)

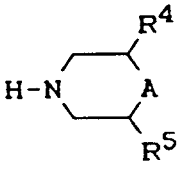

(VI)

in which

$R^4$, $R^5$ and A    have the abovementioned meaning,

if appropriate in the presence of an acid acceptor and if appropriate in the presence of a diluent,

or

b) the pyrimidin-4-yl carbamates which can be obtained by processes (a-$\alpha$) and (a-$\beta$), of the formula (Ia)

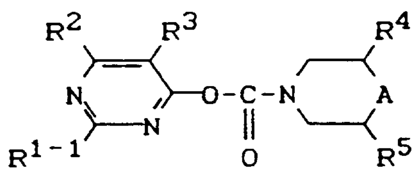

(Ia)

in which

$R^2$, $R^3$, $R^4$, $R^5$ and A    have the abovementioned meaning and

$R^{1-1}$    represents alkylthioalkyl having 1 to 4 carbon atoms in the individual alkyl moieties,

are reacted with 1 to 3.5 mol of an oxidant per mole of carbamate (Ia), if appropriate in the presence of diluents and, if appropriate, in the presence of a catalyst, and the compounds of the general formula (I) are isolated.

**2.** Process for the preparation of pyrimidin-4-yl carbamates of the formula (I) according to Claim 1, characterized in that, in the formulae (I), (Ia), (II), (III), (IV), (V), and (VI) respectively,

$R^1$    represents methoxy, ethoxy, methylthio, ethylthio, methylthiomethyl, ethylthiomethyl, 2-methylthioethyl, methylsulphinylmethyl, ethylsulphinylmethyl, 2-methylsulphinylethyl, methylsulphonylmethyl, ethylsulphonylmethyl, 2-methylsulphonylethyl, dimethylamino or diethylamino,

$R^2$    represents hydrogen, methyl or ethyl,

$R^3$    represents hydrogen, methyl, ethyl, isopropyl, methoxy or ethoxy or

$R^2$ and $R^3$    together represent a double-linked alkanediyl radical having 3 or 4 carbon atoms,

$R^4$    represents hydrogen or methyl,

$R^5$    represents hydrogen or methyl and

A    represents oxygen, sulphur or the radical

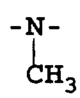

or

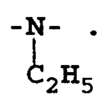 .

**3.** Pesticides, characterized in that they contain at least one pyrimidin-4-yl carbamate of the formula (I) according to Claim 1.

**4.** Insecticidal agents, characterized in that they contain at least one pyrimidin-4-yl carbamate of the formula (I) according to Claim 1.

**5.** Method of controlling animal pests, in particular insects, characterized in that pyrimidin-4-yl carbamates of the formula (I) according to Claim 1 are allowed to act on animal pests and/or their environment.

**6.** Use of pyrimidin-4-yl carbamates of the formula (I) according to Claim 1 for controlling animal pests, in particular insects, with the exception of the uses which cannot be protected in accordance with Art. 52-(4) EPC.

**7.** Process for the preparation of agents against animal pests, characterized in that pyrimidin-4-yl carbamates of the formula (I) according to Claim 1 are mixed with extenders and/or surface-active agents.

**Revendications**
**Revendications pour les Etats contractants suivants : BE, CH, DE, DK, FR, GB, IT, LI, NL**

**1.** Esters d'acides pyrimidine-4-yl-carbamiques de formule I

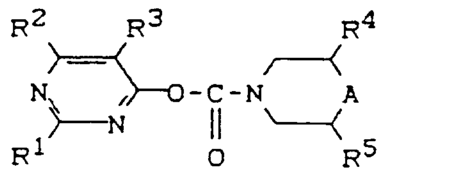

(I)

dans laquelle

| | |
|---|---|
| $R^1$ | représente un groupe alcoxy en $C_1$-$C_4$, alkylthio en $C_1$-$C_4$, alkylthioalkyle contenant 1 à 4 atomes de carbone dans chacune des parties alkyle, alkylsulfinylalkyle contenant 1 à 4 atomes de carbone dans chacune des parties alkyle, alkylsulfonylalkyle contenant 1 à 4 atomes de carbone dans chacune des parties alkyle et dialkylamino contenant 1 à 3 atomes de carbone dans chacune des parties alkyle, |
| $R^2$ | représente l'hydrogène ou un groupe alkyle en $C_1$-$C_4$, |
| $R^3$ | représente l'hydrogène, un groupe alkyle en $C_1$-$C_4$ ou alcoxy en $C_1$-$C_4$, ou bien |
| $R^2$ et $R^3$ | représentent ensemble un groupe alcane-diyle à chaîne droite ou ramifiée en $C_2$-$C_5$ relié aux deux endroits indiqués, |
| $R^4$ | représente l'hydrogène ou un groupe alkyle en $C_1$-$C_3$, |
| $R^5$ | représente l'hydrogène ou un groupe alkyle en $C_1$-$C_3$, et |
| A | représente l'oxygène, le soufre ou un groupe |

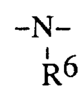

dans lequel
$R^6$ représente un groupe alkyle en $C_1$-$C_3$.

**2.** Esters d'acides pyrimidine-4-yl-carbamiques de formule I selon revendication 1,
dans laquelle

| | |
|---|---|
| $R^1$ | représente un groupe méthoxy, éthoxy, méthylthio, éthylthio, méthylthiométhyle, éthylthiométhyle, 2-méthylthioéthyle, méthylsulfinylméthyle, éthylsulfinylméthyle, 2-méthylsulfinyléthyle, méthylsulfonylméthyle, éthylsulfonylméthyle, 2-méthylsulfonyléthyle, diméthylamino et diéthylamino, |
| $R^2$ | représente l'hydrogène, un groupe méthyle ou éthyle, |
| $R^3$ | représente l'hydrogène, un groupe méthyle, éthyle, isopropyle, méthoxy ou éthoxy, ou bien |
| $R^2$ et $R^3$ | représentent ensemble un groupe alcane-diyle en $C_3$ ou $C_4$ relié aux deux endroits indiqués, |
| $R^4$ | représente l'hydrogène ou un groupe méthyle, |
| $R^5$ | représente l'hydrogène ou un groupe méthyle, et |
| A | représente l'oxygène, le soufre ou un groupe |

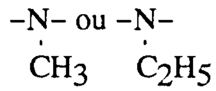

3. Procédé de préparation des esters d'acides pyrimidine-4-yl-carbamiques de formule I de la revendication 1,
caractérisé en ce que :
a) on fait réagir des dérivés de la 4-hydroxypyrimidine de formule II

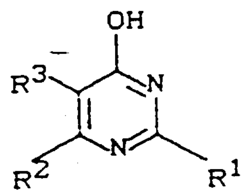

(II)

dans laquelle $R^1$, $R^2$ et $R^3$ ont les significations indiquées dans la revendication 1, ou un sel de métal alcalin, de métal alcalino-terreux ou d'ammonium correspondant,
($\alpha$) avec des chlorures d'acides carbamiques de formule III

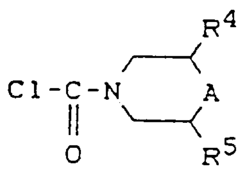

(III)

dans laquelle $R^4$, $R^5$ et A ont les significations indiquées dans la revendication 1, éventuellement en présence d'un accepteur d'acide et éventuellement en présence d'un diluant, ou bien
($\beta$) avec le phosgène de formule IV

$COCl_2$ (IV)

éventuellement en présence d'un accepteur d'acide et éventuellement en présence d'un diluant, ce qui donne les chloroformiates de pyrimidyle de formule V

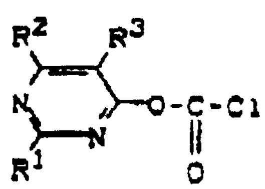

(V)

dans laquelle $R^1$, $R^2$ et $R^3$ ont les significations indiquées dans la revendication 1, qu'on fait réagir, éventuellement après les avoir isolés, avec des amines de formule générale VI

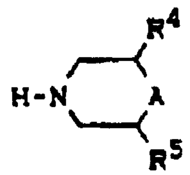

(VI)

dans laquelle $R^4$, $R^5$ et A ont les significations indiquées dans la revendication 1, éventuellement en présence d'un accepteur d'acide et éventuellement en présence d'un diluant, ou bien

b) on fair réagir les esters d'acides pyrimidine-4-yl-carbamiques obtenus par les procédés (a-$\alpha$) et (a-$\beta$), de formule Ia

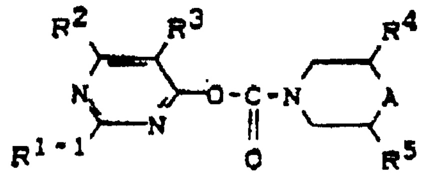

(Ia)

dans laquelle
$R^2$, $R^3$, $R^4$, $R^5$ et A ont les significations indiquées dans la revendication 1, et
$R^{1-1}$ représente un groupe alkylthioalkyle contenant 1 à 4 atomes de carbone dans chacune des parties alkyles,
avec 1 à 3,5 mol d'un agent oxydant par mole de l'ester carbamique Ia, éventuellement en présence de diluants et éventuellement en présence d'un catalyseur, et on isole les composés de formule générale I.

**4.** Produit parasiticide, caractérisé en ce qu'il contient au moins un ester d'acide pyrimidine-4-yl-carbamique de formule I de la revendication 1.

**5.** Produit insecticide, caractérisé en ce qu'il contient au moins un ester d'acide pyrimidine-4-yl-carbamique de formule I de la revendication 1.

**6.** Procédé pour combattre des parasites animaux, en particulier des insectes, caractérisé en ce que l'on fait agir sur les parasites et/ou leur habitat des esters pyrimidine-4-yl-carbamiques de formule I de la revendication 1.

**7.** Utilisation des esters d'acides pyrimidine-4-yl-carbamiques de formule I de la revendication 1, pour la lutte contre les parasites animaux, en particulier des insectes, à l'exception des utilisations qui, en raison de l'article 52(4) de la CBE ne sont pas brevetables.

**8.** Procédé de préparation de produits pour combattre les parasites animaux, caractérisé en ce que l'on mélange des esters d'acides pyrimidine-4-yl-carbamiques de formule I selon revendication 1 avec des diluants et/ou des agents tensioactifs.

**Revendications pour l'Etat contractant suivant : ES**

1. Procédé de préparation des esters d'acides pyrimidine-4-yl-carbamiques de formule I

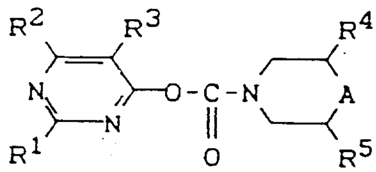

(I)

dans laquelle

| | |
|---|---|
| $R^1$ | représente un groupe alcoxy en $C_1$-$C_4$, alkylthio en $C_1$-$C_4$, alkylthioalkyle contenant 1 à 4 atomes de carbone dans chacune des parties alkyle, alkylsulfinylalkyle contenant 1 à 4 atomes de carbone dans chacune des parties alkyle, alkylsulfonylalkyle contenant 1 à 4 atomes de carbone dans chacune des parties alkyle et dialkylamino contenant 1 à 3 atomes de carbone dans chacune des parties alkyle, |
| $R^2$ | représente l'hydrogène ou un groupe alkyle en $C_1$-$C_4$, |
| $R^3$ | représente l'hydrogène, un groupe alkyle en $C_1$-$C_4$ ou alcoxy en $C_1$-$C_4$, ou bien |
| $R^2$ et $R^3$ | représentent ensemble un groupe alcane-diyle à chaîne droite ou ramifiée en $C_2$-$C_5$ relié aux deux endroits indiqués, |
| $R^4$ | représente l'hydrogène ou un groupe alkyle en $C_1$-$C_3$, |
| $R^5$ | représente l'hydrogène ou un groupe alkyle en $C_1$-$C_3$, et |
| A | représente l'oxygène, le soufre ou un groupe |

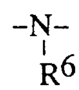

dans lequel

$R^6$ représente un groupe alkyle en $C_1$-$C_3$,

caractérisé en ce que

a) on fait réagir des dérivés de la 4-hydroxypyrimidine de formule II

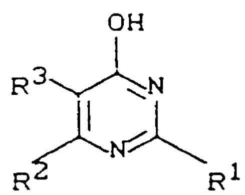

(II)

dans laquelle $R^1$, $R^2$ et $R^3$ ont les significations définies ci-dessus,
ou un sel de métal alcalin, de métal alcalino-terreux ou d'ammonium correspondant,

(α) avec des chlorures d'acides carbamiques de formule III

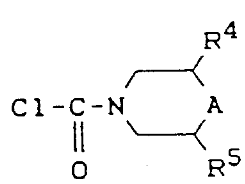

(III)

dans laquelle $R^4$, $R^5$ et A ont les significations définies ci-dessus,
éventuellement en présence d'un accepteur d'acide et éventuellement en présence d'un diluant, ou bien
($\beta$) avec le phosgène de formule IV

$COCl_2$ (IV)

éventuellement en présence d'un accepteur d'acide et éventuellement en présence d'un diluant, ce qui donne les chloroformiates de pyrimidyle de formule V

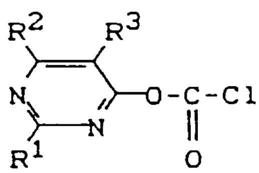

(V)

dans laquelle $R^1$, $R^2$ et $R^3$ ont les significations définies ci-dessus, qu'on fait réagir, éventuellement après les avoir isolés, avec des amines de formule générale VI

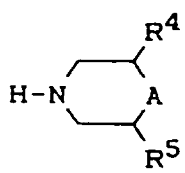

(VI)

dans laquelle $R^4$, $R^5$ et A ont les significations définies ci-dessus, éventuellement en présence d'un accepteur d'acide et éventuellement en présence d'un diluant, ou bien
b) on fait réagir les esters d'acides pyrimidine-4-yl-carbamiques obtenus par les procédés (a-$\alpha$) et (a-$\beta$), de formule Ia

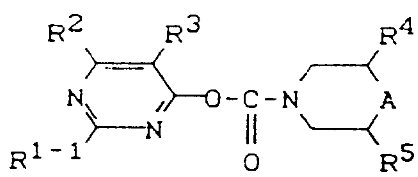

(Ia)

dans laquelle
$R^2$, $R^3$, $R^4$, $R^5$ et A ont les significations définies ci-dessus, et $R^{1-1}$ représente un groupe alkylthioalkyle contenant 1 à 4 atomes de carbone dans chacune des parties alkyles,
avec 1 à 3,5 mol d'un agent oxydant par mole de l'ester carbamique Ia, éventuellement en présence de diluants et éventuellement en présence d'un catalyseur, et isole les composés de formule générale I.

**2.** Procédé de préparation des esters pyrimidine-4-yl-carbamiques de formule I de la revendication 1, caractérisé en ce que, dans les formules I, Ia, II, III, IV, V et VI,

$R^1$ représente un groupe méthoxy, éthoxy, méthylthio, éthylthio, méthylthiométhyle, éthylthiométhyle, 2-méthylthioéthyle, méthylsulfinylméthyle, éthylsulfinylméthyle, 2-méthylsulfinyléthyle, méthylsulfonylméthyle, éthylsulfonylméthyle, 2-méthylsulfonyléthyle, diméthylamino et diéthylamino,
$R^2$ représente l'hydrogène, un groupe méthyle ou éthyle,
$R^3$ représente l'hydrogène, un groupe méthyle, éthyle, isopropyle, méthoxy ou éthoxy, ou

bien

$R^2$ et $R^3$ représentent ensemble un groupe alcane-diyle en $C_3$ ou $C_4$ relié aux deux endroits indiqués,

$R^4$ représente l'hydrogène ou un groupe méthyle,

$R^5$ représente l'hydrogène ou un groupe méthyle, et

A représente l'oxygène, le soufre ou un groupe

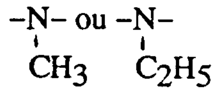
ou .

3. Produit parasiticide, caractérisé en ce qu'il contient au moins un ester d'acide pyrimidine-4-yl-carbamique de formule I de la revendication 1.

4. Produit insecticide, caractérisé en ce qu'il contient au moins un ester d'acide pyrimidine-4-yl-carbamique de formule I de la revendication 1.

5. Procédé pour combattre des parasites animaux, en particulier des insectes, caractérisé en ce que l'on fait agir sur les parasites et/ou leur habitat des esters pyrimidine-4-yl-carbamiques de formule I de la revendication 1.

6. Utilisation des esters d'acides pyrimidine-4-yl-carbamiques de formule I de la revendication 1, pour la lutte contre les parasites animaux, en particulier des insectes, à l'exception des utilisations qui, en raison de l'article 52(4) de la CBE ne sont pas brevetables.

7. Procédé de préparation de produits pour combattre les parasites animaux, caractérisé en ce que l'on mélange des esters d'acides pyrimidine-4-yl-carbamiques de formule I selon revendication 1 avec des diluants et/ou des agents tensioactifs.